# EUROPEAN PATENT SPECIFICATION

(11) **EP 1 833 434 B1**
(45) Date of publication and mention of the grant of the patent: **23.04.2008**
(21) Application number: 05807180.4
(22) Date of filing: 21.11.2005
(51) Int. Cl.: A61F 5/14, A61H 39/04

(54) **PLANTAR FOR VERTICAL PROPRIOCEPTIVE, EXTEROCEPTIVE, PRESSOCEPTIVE AND/OR REFLEXOGEN STIMULATION**
PLANTAR FÜR VERTIKALE PROPRIOZEPTIVE, EXTEROZEPTIVE, PRESSOZEPTIVE UND/ODER REFLEXOGENE STIMULIERUNG
DISPOSITIF PLANTAIRE POUR STIMULATION PROPRIOCEPTIVE, EXTEROCEPTIVE, PRESSOCEPTIVE ET/OU REFLEXOGENE VERTICALE

(30) Priority: 23.11.2004 IT RM20040573
(43) Date of publication of application: 19.09.2007
(73) Proprietor: Neuroreflex Ltd., Dublin 1 (IE)
(72) Inventor: FUSCO, Maria Antonietta KS It. s.a.s. di A.Mario, 83100 Avellino AV (IT)
(74) Representative: Papa, Elisabetta
(86) International application number: PCT/IB2005/053839
(87) International publication number: WO 2006/056931

(56) References cited:
- WO-A-01/03536
- WO-A-03/063630
- US-A- 4 109 661
- US-A- 4 345 387

## Description

The present invention relates to a plantar of the kind suitable to be used for the proprioceptive, exteroceptive, pressoceptive and/or reflexogen stimulation of the foot sole for correcting muscle-skeletal, myo-facial and/or vascular disturbances.

As it is known, the plantar reflexotherapy consists in stimulating determined points of the foot sole, substantially corresponding to the muscle insertions of the so-called intrinsic muscles for maintaining the plantar archs, with which stimulation it is possible affecting the general physiology of the human body for therapeutic purposes.

In particular, the therapeutic correction of the ascending postural anomalies is possible.

With purpose-conceived plantars it is possible carrying out said stimulation for many hours without disturbing the patient, by simply wearing shoes wherein the plantar has been indeed inserted or incorporated.

Different kinds of plantars are known which aim at performing the above-mentioned stimulation of selected areas of the foot sole for reflexological purposes, for example for improving or correcting the person's deambulation or position.

Nevertheless, the known plantars generally have stimulation members, such as for example swellings, all of the same kind and therefore they are apt to exert a single and undifferentiated action onto the various areas and sub-areas of the foot sole, thus not resulting to be selective with respect to the kind of specific stimulation which can be more suitable for each single area or area portion of the foot sole and for each patient. Due to this, the known plantars do not result to be wholly satisfying in terms of obtainable therapeutic results.

US 4 345 387 discloses an insole having a plurality of protrusions in the form of air pockets. Such protrusions may have different height and provide a cushioning and support function.

Therefore, the technical problem posed and solved by the present invention is to provide a plantar for proprioceptive, exteroceptive, pressoceptive and/or reflexogen stimulation of the foot sole allowing to overcome to the drawbacks mentioned above with reference to the known art.

Such a problem is solved by a plantar according to claim 1.

Preferred features of the present invention are shown in the claims depending therefrom.

The present invention provides some important advantages. The main advantage consists in that the plantar of the invention, having at least two kinds of different stimulating elastic members placed at the same stimulation area - and possibly combined therein - or at different areas, allows obtaining a specific and selective stimulation and support. In particular, in the present context under "substantially different" stimulation members it is to be meant that such members have different elastic deformability and/or stimulation properties.

Furthermore, the plantar exerts a pressure always in vertical direction so as to be able to act onto the tone of the antigravitational system of the whole organism.

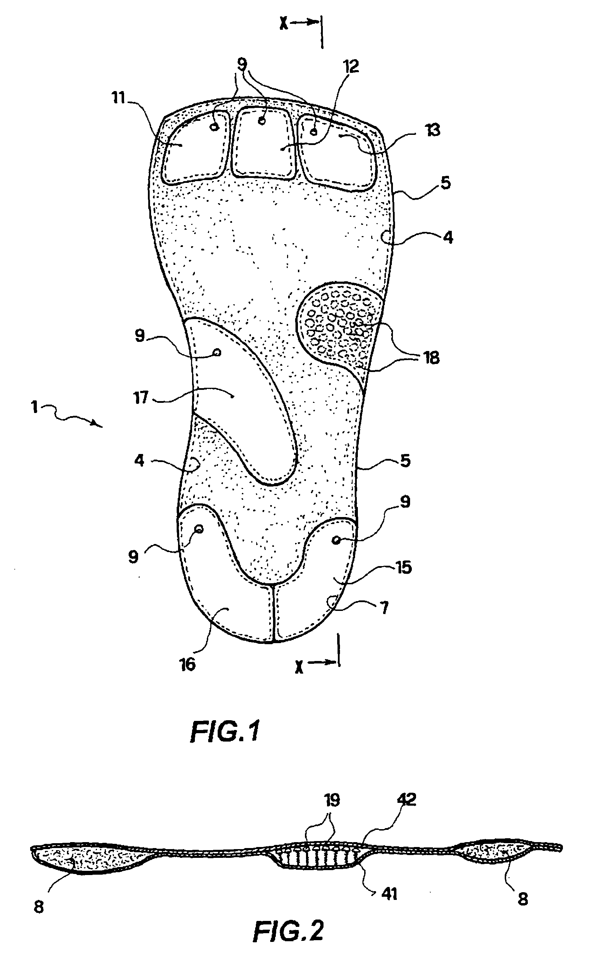

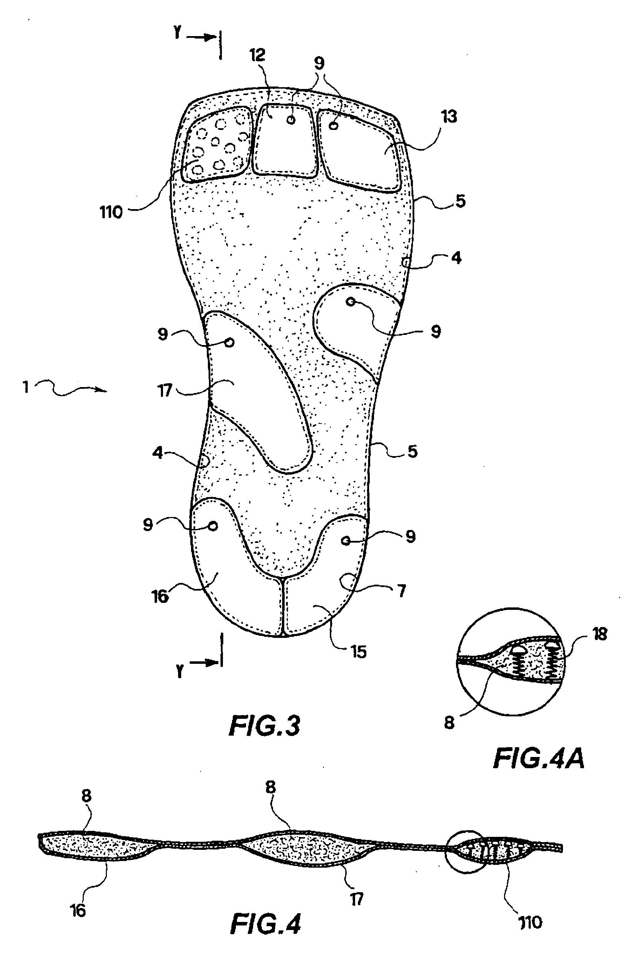

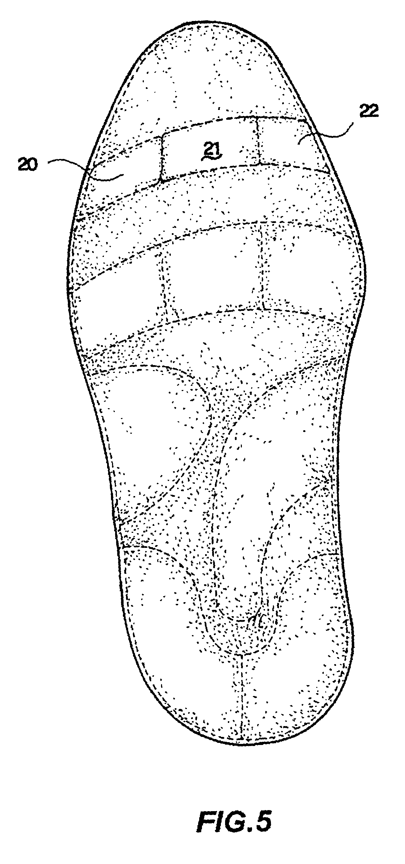

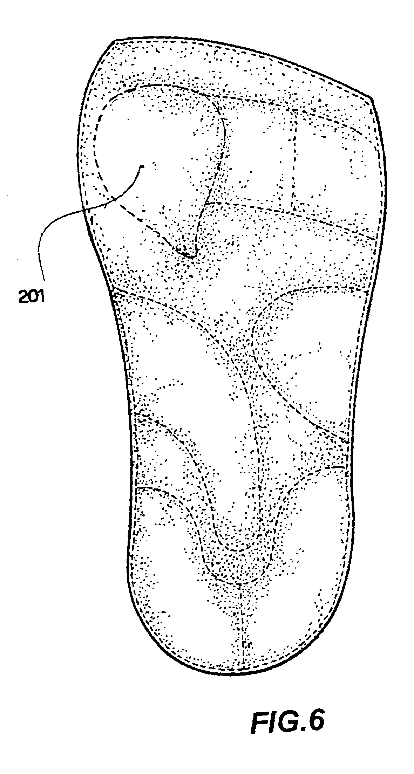

Other advantages, features and application modes of the present invention will be evident from the following detailed description of some embodiments, shown by way of example and not for limitative purpose. The figures of the enclosed drawings will be referred to, wherein:
■ figure 1 shows a plan view of a first embodiment of the plantar according to the present invention;
■ figure 2 shows a cross-sectional view of the plantar of figure 1, carried out according to the line X-X of the latter figure;
■ figure 3 shows a plan view of a second embodiment of the plantar according to the present invention;
■ figure 4 shows a cross-sectional view of the plantar of figure 3, carried out according to line X-X of the latter figure;
■ figure 4A shows an enlarged detail of the plantar of figure 4;
■ figure 5 shows a plan view of a third embodiment of the plantar according to the present invention; and
■ figure 6 shows a plan view of a fourth embodiment of the plantar according to the present invention.

By firstly referring to figures 1 and 2, a plantar for the stimulation of the foot sole according to a first embodiment of the invention is globally designated with 1. The plantar 1 is of the kind apt to be removably extracted and introduced into a shoe.

The plantar 1 is implemented so as to be able to perform a proprioceptive, exteroceptive, pressoceptive and/or reflexogen stimulation for correcting the muscle-skeletal, myo-facial and/or vascular disturbances of the person.

The plantar 1 preferably comprises a first lower layer 2 apt to contact the inner sole of the shoe and a second upper layer 3 apt to contact the person's foot sole, which layers 2 and 3 have the same shape and sizes and they are arranged overlapped and coupled.

The first layer 2, thin and preferably with a thickness equal to about 1 mm, is preferably made of non-toxic rubber and substantially not allergenic material, having long-lasting appropriate peculiarities. As rubber material, a material with natural origin and in particular Pará rubber or regenerated and elastized natural leather is preferred. Of course, a person skilled in the art could appreciate that any other kind of not allergenic material with equivalent mechanical properties could be used. In particular, synthetic rubbers, for example silicone rubbers, could be used.

The second layer 3, thin too, is made of a material soft to the touch suitable for the foot sole, in particular of the kind which to the touch can be assimilated to the chamois. Also in this case a tissue of natural origin, and in particular alcantara, is preferred. Embodiment variants can then provide the use of a tissue or a laminated sheet of a material suitable to guarantee the foot transpiration and a sufficient contact comfort.

The first layer 2 and the second layer 3 are glued therebetween at appropriate areas, as it will be better explained hereinafter. The structure of the plantar 1 is reinforced by a peripheral seam 4, continuous along the edge 5 of the plantar 1.

The choice of the materials and of the structure mentioned above makes the plantar very resistant. This is very important since this type of plantars is subjected to strong wear, mostly due to the foot's sweating and to the cross stresses determined by the pressure exerted by the foot during deambulation, which wear can negatively affect the stimulating capability of the plantar, that, on the contrary, must be kept unaltered for a sufficiently long period of time.

According to the invention, the plantar 1 then includes a plurality of stimulation members placed at proprioceptors, exteroceptors, pressoceptors and reflexological points of the foot sole.

As it is known to the persons skilled in the art and as reminded in the introduction, such proprioceptors, exteroceptors, pressoceptors and reflexological points, of superficial, articular and deep kind, are substantially located at the muscle insertions of the foot sole.

Still according to the invention, the stimulation members are of two substantially different kinds.

A first kind of stimulation members consists in elastically deformable swellings formed between the first and the second layer 2 and 3. To this purpose, the plantar 1 is equipped with a plurality of cells 6 at which the first and the second layer 2 and 3 have not the adhesive keeping them together. The periphery of the cells 6 can be defined and reinforced by respective linear seams 7.

In the present embodiment, each cell 6 is filled-up or apt to be filled up with elastic discreet particles 8 having a substantially irregular shape with facets and edges and implemented in substantially not allergenic rubber. The particles 8, which can be produced by properly cutting in small cubes the same sheet of Pará rubber utilized for packing the first layer 2 of the plantar 1, preferably each one has cross sizes (thickness) of about 1 mm.

Preferably, the filling material of the cells is of the kind known under the commercial name of Biotens®. Alternatively, they can be filled up with natural caoutchouc.

Therefore, the cells 6 filled-up with particles 8 implement the swellings mentioned above.

In the present embodiment, each cell 6 has an opening 9, arranged near the respective linear seam, to allow its filling-up by means of a cannula or analogous device. The openings 9 can be then closed again once the filling thereof has ended.

The cells 6 can remain empty until, based upon medical prescriptions, they are filled-up with an adequate quantity of elastic particles 8. Subsequently, the openings 9 can be closed again to prevent the particles 8 from going out of their seat.

The thickness of each swelling, which is implemented due to the filling up with the particles and to the natural elasticity of the alcantara and of the rubber, is preferably about 3 mm.

A person skilled in the art will understand that, as alternative or in association with said discreet particles 8, each cell 6 can be filled-up with any other kind of elastic material, for example a material like gel, which results easy to be manipulated and inserted into the cells, or with silicone foils, preferably with a thickness of about 2-3 mm, or still with silicone or other elastic granulated material with regular or irregular shape.

The shape, the thickness and the elasticity of each swelling is pre-determined for a correct reflexotherapeutic stress of the proprioceptors, exteroceptors, pressoceptors and reflexological points in general of the foot sole. In particular, the filling-up of the cells 7 is carried out based upon therapeutic indications related to the patient who has to wear the plantar 1.

In the present embodiment, each plantar 1 has six elastic swellings, with different shape according to the position thereof, corresponding to respective proprioceptive and pressoceptive areas of the foot sole, in particular a first elastic swelling 11 at the big toe's adductor, a second swelling 12 for the big toe's short flexor, a third swelling 13 for the short flexor of the fifth toe, a fourth swelling 15 for the supinator wedge, a fifth swelling 16 for the pronator wedge and a sixth swelling 17 for the big toe's adductor/adductor of the 5th toe.

The second type of stimulation members consists in one or more compression helical springs 18 which in the present example are arranged at the adductor of the fifth toe and extend in the direction substantially orthogonal to the foot sole. Such springs are represented very schematically in figure 1 and in greater detail in figure 2. A fastening between the two layers of the plantar, by means of gluing lines and/or peripheral seams, can be provided only at the periphery of such area at which the helical springs are arranged, as schematically shown in figure 1, so as to provide a containment casing or cell for the elastic members 18 arranged in such area.

Each spring 18 has a first end 41 fastened onto the lower layer 2 and a second end 42 apt to act onto the foot sole. To this purpose, at such second end 42 each elastic member has means 19 for coupling with the foot sole, which in the present embodiment is implemented by means of a member substantially shaped like a half-sphere, a dome or a drop and preferably made of an elastic material such as, for example, silicone, caoutchouc or Pará rubber. In the case mentioned above, wherein also the springs 18 are encompassed in a casing, the contact between the means 19 and the foot sole will be mediated by the upper layer 3 of the plantar 1.

Still based upon a preferred embodiment, the coupling means 19 has a height comprised in a range of about 1-1.5 mm and a plan width of about (2x2) mm.

The coupling means 19 makes comfortable the rest of the foot sole onto the plantar and it allows the pressure wave to propagate to an area of the proprioceptive, exteroceptive, pressoceptive and/or reflexogen areas of the foot sole without creating decubiti or irritations.

Based upon a preferred embodiment, the main body of the elastic members comprised between the two ends 41 and 42 is made of a fibro-elastic material such as, for example, carbon fibre or hardened steel with controlled deflection, that is specific for the weight thereto it has to be subjected and then for the anthropometric features of the patient.

The stiffness and/or the height of the elastic members 18 can also be differentiated in different areas of the foot sole. In particular, it can be provided that the height and the stiffness of the elastic members 18 be greater at the centre of said corresponding area of the adductor of the fifth toe.

It will be understood that, however, the shape, the height and the level of stiffness of each elastic member 18 can be selected so as to obtain a correct reflexotherapic stress of the proprioceptors, exteroceptors, pressoceptor and reflexological points in general of the foot sole in each specific patient and for each specific pathology. Such parameters are selected based upon therapeutical indications and stimulating needs related to the patient which has to wear the plantar 1.

Embodiment variants can then provide cells and springs arranged in positions corresponding to any other point of exteroceptive, pressoceptive and reflexological stimulation of the foot sole, in association or not with the seven proprioceptive points mentioned above. Furthermore, the position of the helicoidal springs and of the swellings could be exchanged to meet specific stimulating needs.

The plantar 1 according to the present invention can be manufactured in various sizes.

By referring also to figure 1, during deambulation the swellings 11, 12, 13, 15, 16 and 17 and the elastic members 18 are squeezed under the person's weight and then little by little, when the load is gradually eliminated, they return into the extended resting position. Preferably, the stiffness of the elastic members 18, or of at least part thereof, is such that, under the maximum load exerted by the person, they reduce their height by about 1/3 or about 1/2.

The plantar of the invention is subject to several embodiments alternative to the one described sofar, some thereof will be described hereinafter by referring to the single elements differentiating them from what sofar illustrated.

First of all, the plantar can be permanently incorporated in a shoe instead of being removable with respect thereto.

Furthermore, the elastic members 18 can provide a different implementation with respect to the one illustrated above, for example by replacing, in all or in some members, the helicoidal springs with spiral compression springs.

Based upon an additional embodiment illustrated in the figures 3, 4 and 4A, the different stimulation members are present simultaneously in one or more areas to be stimulated. In particular, in the example represented in said figures in the corresponding area of the insertion of the big toe's adductor muscle a swelling 110 is provided, containing both the particles mentioned above with irregular shape 8 and the helicoidal springs 18.

In general, as far as the areas which can need or benefit from a double stimulation carried out both with vertically pushing springs and with elastic particles are concerned, there are also the big toe's short flexor, since this is the area with greater articular overload of the forefoot, both during deambulation and in the erect position, the insertion of the big toe's adductor and the wedges. As far as the big toe's adductor and the medial wedge are concerned in particular, said combined stimulation is useful in presence of flatfeet of 2nd or 3rd degree. As far as the adductor of the fifth toe and the side wedge are concerned, said combined stimulation is useful if there are hollow feet and club-feet.

Furthermore, in some particular situations of altered three-dimensional architecture of the three archs of the foot sole, the stimulation of intrinsic muscles not only of the elastic kind, but also of the elasto-pressing kind with vertical course from the bottom to the top, such as the one provided by means of the springs 18, is necessary. This is dictated by the vault-like particular structure of our feet, which should have three archs and keep them also in the erect posture. As it is well known to the persons skilled in the art, the three archs are: a front transverse arch going from the metatarsal-phalangeal articulation of the first toe to the metatarsal-phalangeal articulation of the fifth toe, a longitudinal medial arch going from the metatarsal-phalangeal articulation of the first toe to the lower tubercle of the heel bone and a longitudinal side arch going from the metatarsal-phalangeal articulation of the fifth toe to the lower tubercle of the heel bone. The most frequent alterations which are met are charged to the maximum salience of the three archs and, in particular, of the insertion areas of the big toe's adductor for the front transverse arch the big toe's adductor for the longitudinal medial arch, the adductor of the fifth toe for the longitudinal side arch.

In some more clearly pathological situations, like the flatness of the third degree with valgus of the backfoot and medial inclination of the ankle's articular rima, or the hollowness of third degree with varus of the backfoot and side inclination of the anckle's articular rima, it is important and necessary for a complete correction not only stimulating the exteroceptive and proprioceptive nervous ends of such areas, but also to be able to exert a mechanical stress, still of elastic kind, bringing back to normality as quickly as possible the axis of the anckle's articulation, apart from the foot muscles.

Then, the possibility of being able to exert at the same time such stimulations in the various points of the foot sole with stimulation members of different kind, as provided in the present invention, will result particularly useful in the situation of anatomical damage of the feet, in particular of the congenital club-feet, in the results of polyomyelitis or sever myasthenia, or in the post-surgical or post-traumatic rehabilitation of the foot.

Furthermore, based upon a third embodiment shown in figure 5, additional stimulating areas arranged under the toes are provided, for example for the therapy of the mallet or griff-like toes or for the realignment. In the present embodiment three additional stimulation members, designated with 20, 21 and 22, respectively, are provided which indeed carry out a stimulation and a support of the toes. Preferably, such three stimulation members are swellings of the already described kind, filled-up with an elastic material preferably as discreet particles with irregular shape, as already illustrated.

Figure 6, then, shows an additional embodiment, similar to the preceding one, but wherein the stimulation member arranged at the big toe, here designated with 201, has a substantially drop-like elongated shape. In this way, such member 201 does not stimulate the big toe's short flexor muscle any more, as in the previous embodiment, on the contrary the tendon of the big toe's long flexor, which is an extrinsic muscle of the foot (in fact, in the foot only the tendon of such muscle is inserted in the metatarso-phalangeal articulation of the first toe and for the whole length of the foot sole).

In particular, the member 201 has the substantially drop-like elongated shape mentioned above, by extending proximally with respect to the big toe itself at the tip side of the drop itself, that is indeed by thinning proximally with respect to the toes.

The member 201, as shown in figure 6, preferably extends in substantially longitudinal direction with respect to the foot itself.

Additional embodiment variants can provide in a same plantar any combination of the stimulation members provided in the different embodiments illustrated sofar. For example, different cells filled-up with different materials can be provided, in case in association with additional stimulation members based upon compression springs, or, even in a same cell or at a same stimulating area, a combination of different filling materials and/or of compression springs with different deformation properties and/or a combination of one or more filling materials with one or more springs.

It will be appreciated that the plantar of the invention is able to provide to the patient an optimum reflexotherapic stimulation. Such stimulation can be kept unaltered for a long period of time even longer the life year.

Furthermore, the plantar does not suffer from particular wear and it is always comfortable to wear. Furthermore, it can be washed and it is easy to be used and maintained.

The materials therewith it is constituted guarantee the not allergenicness and the non-toxicity of the plantar.

Furthermore, the plantar 1 guarantees a great comfort for a long period of time, without reducing the therapeutic properties with its continuous use.

The present invention has been sofar described by referring to preferred embodiments. It is to be meant that other embodiments belonging to the same inventive core may exist, all comprised within the protection scope of the herebelow reported claims.

## Claims

1. A plantar (1) apt to the proprioceptive, exteroceptive, pressoceptive and/or reflexogen stimulation for correcting muscle-skeletal, myo-facial and/or vascular disturbances of the foot sole, comprising a plurality of stimulation members (11-13, 16-17, 18) arranged at selected areas of the foot sole to carry out said stimulation by deforming elastically during the subject's deambulation,
wherein said stimulation members are at least of two different kinds, each kind having elastic deformability and/or stimulating properties different from the other, **characterised in that** a first kind of stimulation members consists of an elastically deformable swelling (11-13, 16-17) and a second kind of stimulation members consists of one or more compression springs (18) extending in a direction substantially orthogonal to the foot sole.

2. The plantar (1) according to claim 1, wherein said selected areas of the foot sole are selected in a group comprising areas corresponding to: big toe's adductor, big toe's short flexor, short flexor of the fifth toe, supinator wedge, pronator wedge and big toe's adductor and respective muscle insertions.

3. The plantar (1) according to claim 1 or 2, comprising a lower layer (2) and an upper layer (3), the latter apt to contact the foot sole, between which at least one cell (6) apt to house one or more of said stimulation members (11-13, 16-17, 18) has been obtained.

4. The plantar (1) according to the preceding claim, wherein said first layer (2) is made of a substantially not allergenic material.

5. The plantar (1) according to the preceding claim, wherein said substantially not allergenic material is a natural material.

6. The plantar (1) according to the preceding claim, wherein said substantially not allergenic material is Pará rubber or regenerated and elasticized natural leather.

7. The plantar (1) according to anyone of claims 3 to 6, wherein said second layer (3) is made of a material soft to the touch.

8. The plantar (1) according to the preceding claim, wherein said material soft to the touch is a natural tissue substantially like chamois to the touch.

9. The plantar (1) according to the preceding claim, wherein said material soft to the touch is alcantara.

10. The plantar (1) according to anyone of claims 3 to 9, wherein said first layer (2) has a thickness of about 1 mm:

11. The plantar (1) according to anyone of the claims 3 to 10, wherein said first (2) and second (3) layer are glued therebetween and they have no adhesive at said at least one cell (6).

12. The plantar (1) according to anyone of claims 3 to 11, wherein the periphery of said at least one cell (6) is defined by a respective peripheral seam (7) between said first (2) and second (3) layer.

13. The plantar (1) according to any of the preceding claims, wherein said swelling (11-13, 16-17) is filled-up or apt to be filled-up with an elastic material formed by discreet elastic particles (8).

14. The plantar (1) according to the preceding claim, wherein said particles (8) have a substantially irregular shape.

15. The plantar (1) according to claim 13 or 14, wherein said particles (8) are made of substantially not allergenic rubber.

16. The plantar according to anyone of the preceding claims, wherein said swelling (11-13, 16-17) is filled-up or apt to be filled-up with an elastic material comprising a material like gel.

17. The plantar (1) according to any one of claims 3 to 12, wherein said or each one of said compression springs (18) has a first end (41) fastened at said lower layer (2) and a second end (41) covered by said upper layer (3).

18. The plantar (1) according to any of the preceding claims, wherein said or groups of said compression springs (18) are received in a respective cell (6).

19. The plantar (1) according to anyone of the preceding claims, comprising a plurality of compression springs (18) the stiffness and/or height of which is differentiated at different areas of the foot sole.

20. The plantar (1) according to the preceding claim, wherein a plurality of compression springs (18) is arranged at at least one of said selected areas and wherein the height and/or the stiffness of said compression springs (18) is greater at the centre of said area.

21. The plantar (1) according to anyone of the preceding claims, wherein said or each compression spring (18) has an upper end (42) apt to act on the foot sole, which upper end (42) comprises a coupling means (5) for coupling with the foot sole itself.

22. The plantar (1) according to the preceding claim, wherein said coupling means (5) has a shape selected in a group comprising a shape substantially like a half-sphere, substantially like a dome and substantially like a drop.

23. The plantar (1) according to claim 21 or 22, wherein said coupling means (5) is made of an elastic material.

24. The plantar (1) according to the preceding claim, wherein said coupling means (5) is made of a material selected in a group comprising silicone, caoutchouc and Pará rubber.

25. The plantar (1) according to anyone of claims 21 to 24, wherein said coupling means (5) has a height comprised in a range of about 1 - 1.5 mm.

26. The plantar (1) according to anyone of claims 21 to 25, wherein said coupling means (5) has a plan width equal to about (2x2) mm.

27. The plantar (1) according to anyone of the preceding claims, wherein said or each compression spring (18) has a stiffness such as to reduce its own height of about 1/3 when squeezed during deambulation.

28. The plantar (1) according to anyone of the preceding claims, wherein said or each compression spring (18) has a stiffness such as to reduce its own height of about 1/2 when squeezed during deambulation.

29. The plantar (1) according to anyone of the preceding claims, wherein said or each compression spring (18) is made of a fibro-elastic material.

30. The plantar (1) according to anyone of the preceding claims, wherein said or each compression spring is a helicoidal spring (18).

31. The plantar (1) according to anyone of the preceding claims, wherein said or each compression spring (18) is a spiral spring.

32. The plantar (1) according to anyone of the preceding claims, wherein at at least one of said selected areas an elastically deformable respective swelling (110) is arranged, also containing at least one compression spring (18) inside thereof.

33. The plantar (1) according to the preceding claim, wherein said combined presence of a swelling (110) and of at least one compression spring (18) is provided at a selected area in a group comprising areas corresponding to: the big toe's short flexor, the big toe's adductor, plantar wedges and adductor of the fifth toe and respective muscle insertions.

34. The plantar (1) according to anyone of the preceding claims, wherein a first kind of stimulation members consists in an elastic swelling having a first kind of filling material, and a second kind of stimulation members consists in an elastic swelling having a second filling material different from the first one.

35. The plantar (1) according to anyone of the preceding claims, which is a plantar which can be removably inserted and extracted from a shoe.

36. The plantar (1) according to anyone of claims 1 to 34, which is a plantar incorporated in a shoe.

37. The plantar (1) according to anyone of the preceding claims, comprising one or more stimulation members (20, 21, 22, 201) arranged at the toes.

38. The plantar (1) according to the preceding claim, wherein said stimulation members (20, 21, 22, 201) arranged at the toes are suitable for the therapy of mallet or griff-like toes or for the realignment.

39. The plantar (1) according to anyone of the preceding claims, comprising at least a stimulation members (201) arranged at the big toe.

40. The plantar (1) according to the preceding claim, wherein said stimulation members (201) arranged at the big toe has a substantially elongated shape.

41. The plantar (1) according to the preceding claim, wherein said stimulation member (201) arranged at the big toe has a sustantially drop-like shape.

42. The plantar (1) according to anyone of claims 39 to 41, wherein said stimulation member (201) arranged at the big toe has a shape thinning in a proximal direction with respect to the toes.

43. The plantar (1) according to anyone of the claims 39 to 42, wherein said stimulation member (201) arranged at the big toe extends mainly in a substantially longitudinal direction with respect to the foot itself.

## Patentansprüche

1. Sohle (1) für die propriozeptive, exterozeptive, pressozeptive und/oder reflexogene Stimulation zum Korrigieren von Muskel-Skelett-, myofaszialen und/oder vaskulären Störungen der Fußsohle, mit mehreren Stimulationsteilen (11-13, 16-17, 18), die in ausgewählten Bereichen der Fußsohle angeordnet sind, um die Stimulation durch elastisches Verformen beim Gehen der Person zu bewirken,
wobei die Stimulationsteile wenigstens von zwei verschiedenen Arten sind, wobei jede Art elastische Verformbarkeit und/oder Stimulationseigenschaften hat, die von denen der anderen verschieden sind,
**dadurch gekennzeichnet, dass** eine erste Art von Stimulationsteilen aus einem elastisch verformbaren Wulst (11-13, 16-17) besteht und dass eine zweite Art von Stimulationsteilen aus einer oder mehreren Druckfedern (18) besteht, die sich in einer Richtung erstrecken, welche zu der Fußsohle im Wesentlichen orthogonal ist.

2. Sohle (1) nach Anspruch 1, wobei die ausgewählten Bereiche der Fußsohle aus einer Gruppe ausgewählt sind, welche Bereiche umfasst, die Folgendem entsprechen: dem Adduktor des großen Zehs, dem kurzen Flexor des großen Zehs, dem kurzen Flexor des fünften Zehs, Supinatorkeil-, Pronatokeil- und dem Adduktor des großen Zehs zugeordnete Muskelinsertionen.

3. Sohle (1) nach Anspruch 1 oder 2, mit einer unteren Schicht (2) und einer oberen Schicht (3), wobei letztere in der Lage ist, die Fußsohle zu berühren, zwischen welchen wenigstens eine Zelle (6) ausgebildet ist, die in der Lage ist, ein oder mehrere von den Stimulationsteilen (11-13, 16-17, 18) aufzunehmen.

4. Sohle (1) nach dem vorhergehenden Anspruch, wobei die erste Schicht (2) aus einem im Wesentlichen nichtallergenen Material hergestellt ist.

5. Sohle (1) nach dem vorhergehenden Anspruch, wobei das im Wesentlichen nichtallergene Material ein natürliches Material ist.

6. Sohle (1) nach dem vorhergehenden Anspruch, wobei das im Wesentlichen nichtallergene Material ein Pará-Gummi oder regeneriertes und e-lastifiziertes Leder ist.

7. Sohle (1) nach einem der Ansprüche 3 bis 6, wobei die zweite Schicht (3) aus einem berührungsweichen Material hergestellt ist.

8. Sohle (1) nach dem vorhergehenden Anspruch, wobei das berührungsweiche Material ein natürliches Gewebe ist, das bei der Berührung im Wesentlichen wie Gämsenleder ist.

9. Sohle (1) nach dem vorhergehenden Anspruch, wobei das berührungsweiche Material Alcantara ist.

10. Sohle (1) nach einem der Ansprüche 3 bis 9, wobei die erste Schicht (2) eine Dicke von etwa 1 mm hat.

11. Sohle (1) nach einem der Ansprüche 3 bis 10, wobei die erste (2) und die zweite (3) Schicht miteinander verklebt sind, aber im Wesentlichen keinen Klebstoff in der wenigstens einen Zelle (6) haben.

12. Sohle (1) nach einem der Ansprüche 3 bis 11, wobei der Umfang der wenigstens einen Zelle (6) durch eine periphere Naht (7) zwischen der ersten (2) und der zweiten (3) Schicht gebildet ist.

13. Sohle (1) nach einem der vorhergehenden Ansprüche, wobei die Wulst (11-13, 16-17) mit einem elastischen Material, welches durch diskrete elastische Partikel (8) gebildet wird, aufgefüllt oder auffüllbar ist.

14. Sohle (1) nach dem vorhergehenden Anspruch, wobei die Partikel (8) eine im Wesentlichen unregelmäßige Form haben.

15. Sohle (1) nach Anspruch 13 oder 14, wobei die Partikel (8) aus im Wesentlichen nichtallergenem Gummi bestehen.

16. Sohle nach einem der vorhergehenden Ansprüche, wobei der Wulst (11-13, 16-17) mit einem elastischem Material, das ein Material wie Gel umfasst, aufgefüllt oder auffüllbar ist.

17. Sohle (1) nach einem der Ansprüche 3-12, wobei die oder jede der Druckfedern (18) ein erstes Ende (41) hat, das an der unteren Schicht (2) befestigt ist, und ein zweites Ende (41), das durch die obere Schicht (3) bedeckt ist.

18. Sohle (1) nach einem der vorhergehenden Ansprüche, wobei die Gruppe oder jede der Gruppen der Druckfedern (18) in einer Zelle (6) aufgenommen ist.

19. Sohle (1) nach einem der vorhergehenden Ansprüche, mit einer Vielzahl von Druckfedern (18), deren Steifigkeit und/oder Höhe in unterschiedlichen Bereichen der Fußsohle unterschiedlich ist.

20. Sohle (1) nach dem vorhergehenden Anspruch, wobei eine Vielzahl der Druckfedern (18) in wenigsten einem der ausgewählten Bereiche angeordnet ist und wobei die Höhe und/oder die Steifigkeit der Druckfedern (18) im Zentrum des Bereiches größer ist.

21. Sohle (1) nach einem der vorhergehenden Ansprüche, wobei die oder jede Druckfeder (18) ein oberes Ende (42) hat, das in der Lage ist, auf die Fußsohle einzuwirken, wobei das obere Ende (42) eine Koppeleinrichtung (5) zum Koppeln mit der Fußsohle selbst aufweist.

22. Sohle (1) nach dem vorhergehenden Anspruch, wobei die Koppeleinrichtung (5) eine Form hat, die aus einer Gruppe ausgewählt ist, welche eine Form im Wesentlichen wie eine Halbkugel, im Wesentlichen wie eine Kuppel und im Wesentlichen wie ein Tropfen umfasst.

23. Sohle (1) nach Anspruch 21 oder 22, wobei die Koppeleinrichtung (5) aus einem elastischen Material besteht.

24. Sohle (1) nach dem vorhergehenden Anspruch, wobei die Koppeleinrichtung (5) aus einem Material besteht, das aus einer Gruppe ausgewählt ist, die Silikon, Kautschuk und Pará-Gummi umfasst.

25. Sohle (1) nach einem der Ansprüche 21 bis 24, wobei die Koppeleinrichtung (5) eine Höhe hat, die in einem Bereich von etwa 1 - 1,5 mm liegt.

26. Sohle (1) nach einem der Ansprüche 21 bis 25, wobei die Koppeleinrichtung (5) im Grundriss eine Breite hat, die etwa gleich (2x2) mm ist.

27. Sohle (1) nach einem der vorhergehenden Ansprüche, wobei die oder jede Druckfeder (18) eine derartige Steifigkeit hat, dass ihre eigene Höhe um etwa 1/3 reduziert wird, wenn sie während des Gehens zusammengedrückt wird.

28. Sohle (1) nach einem der vorhergehenden Ansprüche, wobei die oder jede Druckfeder (18) eine derartige Steifigkeit hat, dass ihre eigene Höhe um etwa 1/2 reduziert wird, wenn sie während des Gehens zusammengedrückt wird.

29. Sohle (1) nach einem der vorhergehenden Ansprüche, wobei die oder jede Druckfeder (18) aus einem elastischen Fasermaterial besteht.

30. Sohle (1) nach einem der vorhergehenden Ansprüche, wobei die oder jede Druckfeder eine Schraubenfeder (18) ist.

31. Sohle (1) nach einem der vorhergehenden Ansprüche, wobei die oder jede Druckfeder (18) eine Spiralfeder ist.

32. Sohle (1) nach einem der vorhergehenden Ansprüche, wobei in wenigstens einem der ausgewählten Bereiche ein elastisch verformbarer Wulst (110) angeordnet ist, der in seinem Inneren ebenfalls wenigstens eine Druckfeder (18) enthält.

33. Sohle (1) nach dem vorhergehenden Anspruch, wobei das kombinierte Vorhandensein eines Wulstes (110) und wenigstens einer Druckfeder (18) in einem ausgewählten Bereich in einer Gruppe vorhanden ist, welche Bereiche umfasst, die Folgendem entsprechen: dem kurzen Flexor des großen Zehs, dem Adduktor des großen Zehs, Sohlenkeilen und dem Adduktor des fünften Zehs und zugeordneten Muskelinsertionen.

34. Sohle (1) nach einem der vorhergehenden Ansprüche, wobei eine erste Art von Stimulationsteilen aus einem elastischen Wulst besteht, der eine erste Art von Füllmaterial hat, und wobei eine zweite Art von Stimulationsteilen aus einem elastischen Wulst besteht, der ein zweites Füllmaterial hat, das von dem des ersten verschieden ist.

35. Sohle (1) nach einem der vorhergehenden Ansprüche, wobei es sich um eine Sohle handelt, die lösbar in einen Schuh eingelegt und aus einem Schuh entnommen werden kann.

36. Sohle (1) nach einem der Ansprüche 1 bis 34, wobei es sich um eine Sohle handelt, die in einen Schuh eingearbeitet ist.

37. Sohle (1) nach einem der vorhergehenden Ansprüche, mit einem oder mehreren Stimulationsteilen (20, 21, 22, 201), die an den Zehen angeordnet sind.

38. Sohle (1) nach dem vorhergehenden Anspruch, wobei die Stimulationsteile (20, 21, 22, 201), die an den Zehen angeordnet sind, für die Therapie von hammer- oder krallenartigen Zehen oder für die Wiederausrichtung geeignet sind.

39. Sohle (1) nach einem der vorhergehenden Ansprüche, wobei wenigstens ein Stimulationsteil (201) an dem großen Zeh angeordnet ist.

40. Sohle (1) nach dem vorhergehenden Anspruch, wobei das Stimulationsteil (201), das an dem großen Zeh angeordnet ist, eine im Wesentlichen langgestreckte Form hat.

41. Sohle (1) nach dem vorhergehenden Anspruch, wobei das Stimulationsteil (201), das an dem großen Zeh angeordnet ist, eine im Wesentlichen tropfenartige Form hat.

42. Sohle (1) nach einem der Ansprüche 39 bis 41, wobei das Stimulationsteil (201), das an dem großen Zeh angeordnet ist, eine Form hat, die in proximaler Richtung in Bezug auf die Zehen dünner wird.

43. Sohle (1) nach einem der Ansprüche 39 bis 42, wobei das Stimulationsteil (201), das an dem großen Zeh angeordnet ist, sich hauptsächlich in einer im Wesentlichen longitudinalen Richtung in Bezug auf den Fuß selbst erstreckt.

## Revendications

1. Dispositif plantaire (1) apte à la stimulation proprioceptive, extéroceptive, pressoceptive et/ou reflexogène destiné à corriger les troubles musculo-squelettiques, myofasciaux et/ou vasculaires de la plante du pied, comprenant une pluralité d'éléments de stimulation (11-13, 16-17, 18) disposés dans des zones sélectionnées de la plante du pied pour effectuer ladite stimulation par déformation élastique pendant la déambulation du sujet,
dans lequel lesdits éléments de stimulation sont au moins de deux catégories différentes, chaque catégorie ayant une déformabilité élastique et/ou des propriétés de stimulation différentes de l'autre, **caractérisé en ce qu'**une première catégorie d'éléments de stimulation se compose d'un gonflement déformable élastiquement (11-13, 16-17) et qu'une seconde catégorie d'éléments de stimulation se compose d'un ou de plusieurs ressorts de compression (18) s'étendant dans une direction sensiblement orthogonale à la plante du pied.

2. Dispositif plantaire (1) selon la revendication 1, dans lequel lesdites zones sélectionnées de la plante du pied sont sélectionnées dans un groupe comprenant des zones correspondant : à l'adducteur du gros orteil, au fléchisseur court du gros orteil, au fléchisseur court du cinquième orteil, au coin supinateur, au coin pronateur et à l'adducteur du gros orteil et aux insertions musculaires respectives.

3. Dispositif plantaire (1) selon les revendications 1 ou 2, comprenant une couche inférieure (2) et une couche supérieure (3), cette dernière pouvant toucher la plante du pied, entre laquelle au moins une cellule (6) apte à loger un ou plusieurs desdits éléments de stimulation (11-13, 16-17, 18) a été obtenue.

4. Dispositif plantaire (1) selon la revendication précédente, dans lequel ladite première couche (2) est faite d'une matière essentiellement anallergisante.

5. Dispositif plantaire (1) selon la revendication précédente, dans lequel ladite matière essentiellement anallergisante est une matière naturelle.

6. Dispositif plantaire (1) selon la revendication précédente, dans lequel ladite matière essentiellement anallergisante est du caoutchouc Para ou du cuir naturel régénéré et élastique.

7. Dispositif plantaire (1) selon l'une quelconque des revendications 3 à 6, dans lequel la seconde couche (3) est faite d'une matière douce au toucher.

8. Dispositif plantaire (1) selon la revendication précédente, dans lequel ladite matière douce au toucher est un tissu naturel essentiellement semblable à la peau de chamois au toucher.

9. Dispositif plantaire (1) selon la revendication précédente, dans lequel ladite matière douce au toucher est de l'alcantara.

10. Dispositif plantaire (1) selon l'une quelconque des revendications 3 à 9, dans lequel ladite première couche (2) a une épaisseur d'environ 1 mm.

11. Dispositif plantaire (1) selon l'une quelconque des revendications 3 à 10, dans lequel lesdites première (2) et seconde (3) couches sont collées entre elles et n'ont aucun adhésif sur au moins une cellule (6).

12. Dispositif plantaire (1) selon l'une quelconque des revendications 3 à 11, dans lequel la périphérie de ladite au moins une cellule (6) est définie par une bordure périphérique respective (7) entre lesdites première (2) et seconde (3) couches.

13. Dispositif plantaire (1) selon l'une quelconque des revendications précédentes, dans lequel ledit gonflement (11-13, 16-17) est rempli ou peut être rempli par une matière élastique formée de discrètes particules élastiques (8).

14. Dispositif plantaire (1) selon la revendication précédente, dans lequel lesdites particules ont une forme essentiellement irrégulière.

15. Dispositif plantaire (1) selon les revendications 13 ou 14, dans lequel lesdites particules (8) sont faites d'un caoutchouc essentiellement anallergisant.

16. Dispositif plantaire selon l'une quelconque des revendications précédentes, dans lequel ledit gonflement (11-13, 16-17) est rempli ou peut être rempli par une matière élastique comprenant une matière semblable à du gel.

17. Dispositif plantaire (1) selon l'une quelconque des revendications 3 à 12, dans lequel lesdits ressorts de compression ou chacun desdits ressorts de compression (18) a une première extrémité (41) attachée à ladite couche inférieure (2) et une seconde extrémité (41) couverte par ladite couche supérieure (3).

18. Dispositif plantaire (1) selon l'une quelconque des revendications précédentes, dans lequel lesdits ressorts de compression ou chacun desdits groupes desdits ressorts de compression (18) sont réceptionnés dans une cellule respective (6).

19. Dispositif plantaire (1) selon l'une quelconque des revendications précédentes comprenant une pluralité de ressorts de compression (18), dont la rigidité et/ou la hauteur est différenciée à différents niveaux de la plante du pied.

20. Dispositif plantaire (1) selon la revendication précédente, dans lequel une pluralité de ressorts de compression (18) est disposée dans au moins une des dites zones sélectionnées et selon lequel la hauteur et/ou la rigidité desdits ressorts de compression (18) est supérieure au centre de ladite zone.

21. Dispositif plantaire (1) selon l'une quelconque des revendications précédente, dans lequel ledit ou chaque ressort de compression (18) a une extrémité supérieure (42) capable d'agir sur la plante du pied, dont l'extrémité supérieure (42) comprend un moyen de couplage (5) destiné à être couplé avec la plante du pied elle-même..

22. Dispositif plantaire (1) selon la revendication précédente, dans lequel ledit moyen de couplage (5) a une forme sélectionnée dans un groupe comprenant une forme ressemblant essentiellement à une demi-sphère, essentiellement à un dôme et essentiellement à une goutte.

23. Dispositif plantaire (1) selon la revendication 21 ou 22, dans lequel ledit moyen de couplage (5) est fait d'une matière élastique.

24. Dispositif plantaire (1) selon la revendication précédente, dans lequel ledit moyen de couplage (5) est fait d'une matière sélectionnée dans un groupe contenant du silicone, du caoutchouc et du caoutchouc Para.

25. Dispositif plantaire (1) selon l'une quelconque des revendications 21 à 24, dans lequel ledit moyen de couplage (5) a une hauteur d'environ 1 à 1,5 mm.

26. Dispositif plantaire (1) selon l'une quelconque des revendications 21 à 25, dans lequel ledit moyen de couplage (5) a une largeur plane égale à environ (2 x 2) mm.

27. Dispositif plantaire (1) selon l'une quelconque des revendications précédentes, dans lequel chaque ressort de compression (18) a une rigidité de manière à réduire sa propre hauteur d'environ 1/3 lorsqu'il est comprimé pendant la déambulation.

28. Dispositif plantaire (1) selon l'une quelconque des revendications précédentes, dans lequel chaque ressort de compression (18) a une rigidité de manière à réduire sa propre hauteur d'environ 1/2 lorsqu'il est comprimé pendant la déambulation.

29. Dispositif plantaire (1) selon l'une quelconque des revendications précédentes, dans lequel ledit ou chaque ressort de compression (18) est fait d'une matière fibro-élastique.

30. Dispositif plantaire (1) selon l'une quelconque des revendications précédentes, dans lequel ledit ou chaque ressort de compression est un ressort hélicoïdal (18).

31. Dispositif plantaire (1) selon l'une quelconque des revendications précédentes, dans lequel ledit ou chaque ressort de compression (18) est un ressort spiral.

32. Dispositif plantaire (1) selon l'une quelconque des revendications précédentes, dans lequel un gonflement respectif élastiquement déformable (110) est disposé sur au moins une des dites zones sélectionnées, contenant aussi au moins un ressort de compression (18) à l'intérieur.

33. Dispositif plantaire (1) selon l'une des revendications précédentes, dans lequel ladite présence combinée d'un gonflement (110) et d'au moins un ressort de compression (18) est possible dans une zone sélectionnée dans un groupe comprenant des zones correspondant : au fléchisseur court du gros orteil, à l'adducteur du gros orteil, aux coins plantaires et à l'adducteur du cinquième orteil et aux insertions musculaires respectives.

34. Dispositif plantaire (1) selon l'une quelconque des revendications précédentes, dans lequel une première catégorie d'éléments de stimulation se compose d'un gonflement élastique ayant une première catégorie de matière de remplissage et une seconde catégorie d'éléments de stimulation se compose d'un gonflement élastique ayant une seconde matière de remplissage différente de la première.

35. Dispositif plantaire (1) selon l'une quelconque des revendications précédentes qui est un dispositif plantaire pouvant être inséré de manière amovible et retiré d'une chaussure.

36. Dispositif plantaire (1) selon l'une quelconque des revendications 1 à 34, qui est un dispositif plantaire incorporé dans une chaussure.

37. Dispositif plantaire (1) selon l'une quelconque des revendications précédentes comprenant un ou plusieurs éléments de stimulation (20, 21, 22, 201) placés aux orteils.

38. Dispositif plantaire (1) selon la revendication précédente, selon lequel lesdits éléments de stimulation (20, 21, 22, 201) placés aux orteils sont adaptés à la thérapie d'orteils en maillet ou en griffe ou pour leur réalignement.

39. Dispositif plantaire (1) selon l'une quelconque des revendications précédentes comprenant au moins un élément de stimulation (201) placé au gros orteil.

40. Dispositif plantaire (1) selon la revendication précédente, selon lequel ledit élément de stimulation (201) placé au gros orteil a une forme sensiblement allongée.

41. Dispositif plantaire (1) selon la revendication précédente, selon lequel ledit élément de stimulation (201) placé au gros orteil a sensiblement la forme d'une goutte.

42. Dispositif plantaire (1) selon l'une quelconque des revendications 39 à 41, selon lequel ledit élément de stimulation (201) placé au gros orteil a une forme s'amincissant dans une direction proximale par rapport aux orteils.

43. Dispositif plantaire (1) selon l'une quelconque des revendications 39 à 42, selon lequel ledit élément de stimulation (201) placé au gros orteil s'étend principalement dans une direction essentiellement longitudinale par rapport au pied lui-même.
